# EUROPEAN PATENT APPLICATION

(11) **EP 1 247 533 A2**
(43) Date of publication of application: **09.10.2002**
(21) Application number: 02251844.3
(22) Date of filing: 14.03.2002
(51) Int. Cl.: A61K 45/06, A61P 25/02

(54) **Combination treatment of multiple sclerosis (MS), other demyelinating conditions and peripheral neuropathy, especially painful neuropathies and diabetic neuropathy**

(30) Priority: 05.04.2001 US 281988 P
(71) Applicant: Pfizer Products Inc., Groton, Connecticut 06340 (US)
(72) Inventor: Howard, Jr, Harry Ralph, Groton, Connecticut 06340 (US)
(74) Representative: Stuart, Peter Graham

(57) **Abstract**

The present invention relates to a combination useful in treating Multiple Sclerosis, other demyelinating disorders and peripheral neuropathy in a mammal comprising a neurotransmitter-inducing or precursor agent in combination with an (SRI) anxiolytic agent or an antidepressant with improvement in efficiency. It also relates to pharmaceutical compositions containing a pharmaceutically acceptable carrier, a neurotransmitter-inducing or precursor agent, and an SRI antidepressant or anxiolytic agent.

## Description

This invention relates to a method of treating Multiple Sclerosis (MS) and other demyelinating conditions and peripheral neuropathy in a mammal by administering to the mammal a Serotonin Reuptake Inhibitor (SRIs) in combination with a neurotransmitter-inducing or precursor agent. It also relates to pharmaceutical compositions containing a pharmaceutically acceptable carrier, a Serotonin Reuptake Inhibitor (SRI) and a neurotransmitter-inducing or precursor agent.

Multiple sclerosis is a common and well-known neurological disorder. It is characterized by episodic patches of inflammation and demyelination which can occur anywhere in the central nervous system (CNS) almost always without any involvement of the peripheral nerves. The occurrence of the patches is disseminated in time and space, hence the older alternative name of disseminated sclerosis. It is believed that the pathogenesis involves local disruption of the blood brain barrier, a local immune and inflammatory response, with consequent damage to myelin and hence to neurons.

Clinically, MS can present in both sexes and at any age. However, its most common presentation is in relatively young adults often with a single focal lesion such as damage to the optic nerve (optic neuritis), an area of anesthesia or paresthesia or muscular weakness. Vertigo, nystagmus double vision, pain, incontinence, cerebellar signs, L'Hermitte's sign (paresthesia or pain in the arms and legs on flexing the neck) and a large variety of less common symptoms may occur. The initial attack is often transient and it may be weeks, months or years before a further attack occurs. Some individuals may have a stable condition, while others may have an unrelenting downhill course ending in complete paralysis. More commonly there is a long series of remissions and relapses, each relapse leaving the patient somewhat worse than before. Relapses may be triggered by stressful events or viral infections. Elevated body temperature almost invariably makes the condition worse whereas a reduced temperature, for example induced by a cold bath, may make the condition better.

There are no satisfactory treatments for MS. Steroids may produce a temporary improvement but any beneficial effect invariably wears off. Recent clinical trials have shown that interferon may somewhat reduce the risk of relapse. However, the effect is modest and most patients still deteriorate.

Pain can originate in damage to the central or peripheral nervous system itself and may persist long after the original cause of the damage has been removed. This type of pain is usually called neuropathic or neuralgic and has many causes. Any form of trauma or other damage to any peripheral nerve or to certain parts of the central nervous system may be followed by prolonged pain which may persist for months, years or decades. The damage may be caused by accidental or surgical injury, by metabolic disturbances such as diabetes or vitamin B12 or other nutrient deficiency, by ischaemia, by radiation, by autoimmune attack, by alcohol, by infections, especially viral infections, particularly with the herpes virus, by tumors, by degenerative diseases, or by unknown factors such as may be operative in trigeminal and other neuralgias. This is by no means an exhaustive list. These types of pain often respond poorly to treatment and patients suffering them have frequently been subjected to trials of many different drugs without success. The most consistent successes are perhaps achieved with antidepressant drugs of various types. A drug for temporal lobe epilepsy, carbamazepine, is sometimes effective in trigeminal neuralgia though not usually in other types of pain in this class.

Any individual who is experiencing pain immediately wants it relieved. Pain from peripheral neuropathy is often poorly responsive to existing methods of treatment and many patients have tried large numbers of drugs without real success. The problems of dealing with such pain have been well described in recent articles such as those by J. W. Scadding, pp 3936 to 3946 in the 3rd edition of the Oxford Textbook of Medicine, Oxford University Press, 1996; 1996; R. G. Kost and S. E. Straus in the New England Journal of Medicine 335:32-42, 1996; and B. S. Galer in Neurology, 45, Supplement 9, S17-S25, 1996.

Diabetic neuropathy may be mild, for example taking the form of "burning" or tingling in the feet or numbness and/or loss of vibration sense in the extremities, especially the feet. Moderate to severe symptoms of neuropathy include pain and spasm in the extremities (painful neuropathy with spasm). Diabetic amyotrophy is indicated by pain over the thigh and loss of quadriceps power, sometimes also loss of power in the lower leg resulting in foot drop. Autonomic neuropathy principally affects the nerves supplying the heart and viscera. Mononeuritis is usually caused by a single peripheral nerve palsy.

Other peripheral neuropathies include the following:
- HIV associated neuropathy;
- B₁₂ - deficiency associated neuropathy;
- cranial nerve palsies;
- drug-induced neuropathy;
- industrial neuropathy;
- lymphomatous neuropathy;
- myelomatous neuropathy;
- multi-focal motor neuropathy;
- chronic idiopathic sensory neuropathy;
- carcinomatous neuropathy;
- acute pain autonomic neuropathy;
- alcoholic neuropathy;
- compressive neuropathy; and
- mono- and poly- neuropathies.

### Summary Of The Invention

The present invention relates to a pharmaceutical composition for the treatment of Multiple Sclerosis (MS), other demyelinating conditions, and peripheral neuropathy comprising: (a) a compound that exhibits activity as a Serotonin Reuptake Inhibitor (SRI), or a pharmaceutically acceptable salt thereof; (b) a neurotransmitter inducing or precursor agent or pharmaceutically acceptable salt thereof; and (c) a pharmaceutically acceptable carrier; wherein the active agents "a" and "b" above are present in amounts that render the composition effective in treating, respectively, Multiple Sclerosis, other demyelinating conditions especially encephalomyelitis and peripheral neuropathy especially painful neuropathies and diabetic neuropathy.

This invention also relates to a method of treating Multiple Sclerosis, other demyelinating conditions and peripheral neuropathy in a mammal comprising administering to said mammal, respectively, an anti-Multiple Sclerosis, anti-demyelinating or anti-peripheral neuropathy effective amount of a pharmaceutical composition comprising: (a) a Serotonin Reuptake Inhibitor (SRI) compound that exhibits activity as, respectively, an anxiolytic or an antidepressant, or a pharmaceutically acceptable salt thereof; (b) a neurotransmitter-inducing or precursor agent or pharmaceutically acceptable salt thereof; and (c) a pharmaceutically acceptable carrier; wherein the active agents "a" and "b" above are present in amounts that render the composition effective in treating, respectively, Multiple Sclerosis, other demyelinating conditions, especially encephalomyelitis and peripheral neuropathy, especially painful neuropathies and diabetic neuropathy with improvement in the efficacy achieved by either component individually.

This invention also relates to a method of treating Multiple Sclerosis, other demyelinating conditions and peripheral neuropathy in a mammal comprising administering to said mammal: (a) a Serotonin Reuptake Inhibitor (SRI) compound that exhibits activity as, respectively, an anxiolytic agent or an antidepressant, or a pharmaceutically acceptable salt thereof; and (b) a neurotransmitter-inducing or precursor agent or pharmaceutically acceptable salt thereof; wherein the active agents "a" and "b" above are present in amounts that render the combination of the two agents effective in treating, respectively, Multiple Sclerosis, other demyelinating conditions and peripheral neuropathy with improvement in the efficacy achieved by either component individually in the treatment of Multiple Sclerosis, other demyelinating conditions, especially encephalomyelitis and peripheral neuropathy especially painful neuropathies and diabetic neuropathy.

It will be appreciated that when using a combination method of the present invention, referred to immediately above, both the neurotransmitter inducing or precursor agent and the (SRI) antidepressant or anti-anxiety agent will be administered to a patient within a reasonable period of time. The compounds may be in the same pharmaceutically acceptable carrier and therefore administered simultaneously. They may be in separate pharmaceutical carriers such as conventional oral dosage forms that are taken simultaneously. The term combination, as used above, also refers to the case where the compounds are provided in separate dosage forms and are administered sequentially. Therefore, by way of example, the (SRI) antidepressant or anxiolytic agent may be administered as a tablet and then, within a reasonable period of time, the neurotransmitter-inducing or precursor agent may be administered either as an oral dosage form such as a tablet or a fast-dissolving oral dosage form. By a "fast dissolving oral formulation" is meant, an oral delivery form which when placed on the tongue of a patient, dissolves within about seconds

The compositions of the present invention that contain a neurotransmitter-inducing or precursor agent and (SRI) antidepressant are useful for the treatment of Multiple Sclerosis, other demyelinating conditions especially encephalomyelitis and peripheral neuropathy especially diabetic neuropathy and painful neuropathy.

The compositions of the present invention are especially useful for the treatment of Multiple Sclerosis, other demyelinating conditions and peripheral neuropathy by compensating for the effects of the nerve damage caused by these conditions. By the use of a combination of a neurotransmitter-inducing or precursor agent and an (SRI) antidepressant or anxiolytic agent in accordance with the present invention, it is possible to treat Multiple Sclerosis, other demyelinating conditions and peripheral neuropathy in patients for whom conventional therapy might not be wholly successful.

The term "treatment", as used herein, refers to reversing, alleviating, inhibiting the progress of, or preventing the disorder or condition to which such term applies, or one or more symptoms of such condition or disorder. The term "treatment", as used herein, refers to the act of treating, as "treating" is defined immediately above.

Examples of Serotonin Reuptake Inhibitors (SRI) that may be used in the methods and pharmaceutical compositions of this invention are compounds of the formula 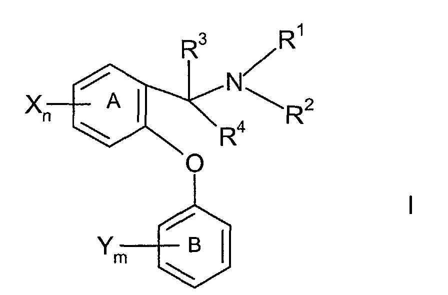 wherein
phenyl ring A and phenyl ring B can each, independently, be replaced by a naphthyl group, and wherein when phenyl ring A is replaced by a naphthyl group, the ethereal oxygen of structure I and the carbon to which R³, R⁴ and NR¹R² are attached, are attached to adjacent ring carbon atoms of the naphthyl group and neither of said adjacent ring carbon atoms is also adjacent to a fused ring carbon atom of said naphthyl group;
n and m are, selected, independently, from one, two and three;
R¹ and R² are selected, independently, from hydrogen (C₁-C₄)alkyl, (C₂-C₄)alkenyl, and (C₂-C₄)alkynyl, or R¹ and R², together with the nitrogen to which they are attached, form a four to eight membered saturated ring containing one or two heteroatoms, including the nitrogen to which R¹ and R² are attached, wherein the second heteroatom, when present, is selected from oxygen, nitrogen and sulfur, and wherein said ring may optionally be substituted at available binding sites with from one to three substituents selected, independently, from hydroxy and (C₁-C₆)alkyl;
R³ and R⁴ are selected, independently, from hydrogen and (C₁-C₄) alkyl optionally substituted with from one to three fluorine atoms, or R³ and R⁴, together with the carbon to which they are attached, form a four to eight membered saturated carbocyclic ring, and wherein said ring may optionally be substituted at available binding sites with from one to three substituents selected, independently, from hydroxy and (C₁-C₆)alkyl;
or R² and R³, together with the nitrogen to which R² is attached and the carbon to which R³ is attached, form a four to eight membered saturated ring containing one or two heteroatoms, including the nitrogen to which R² is attached, wherein the second heteroatom, when present, is selected from oxygen, nitrogen and sulfur, and wherein said ring may optionally be substituted at available binding sites with from one to three substituents selected, independently, from hydroxy and (C₁-C₆)alkyl;
each X and each Y is selected, independently, from hydrogen, halo (i.e., chloro, fluoro, bromo or iodo), (C₁-C₄)alkyl optionally substituted with from one to three fluorine atoms, (C₁-C₄)alkoxy optionally substituted with from one to three fluorine atoms, cyano, nitro, amino, (C₁-C₄)alkylamino, di-[(C₁-C₄)alkyl]amino, NR⁵(C=O)(C₁-C₄)alkyl wherein R⁵ is hydrogen or (C₁-C₆)alkyl, and SOₚ(C₁-C₆)alkyl wherein p is zero, one or two; and
with the proviso that: (a) no more than one of NR¹R², CR³R⁴ and R²NCR³ can form a ring; and (b) at least one X must be other than hydrogen when (i) R³ and R⁴ are both hydrogen, (ii) R¹ and R² are selected, independently, from hydrogen and (C₁-C₄)alkyl, and (iii) ring B is mono- or disubstituted with, respectively, one or two halo groups;
and the pharmaceutically acceptable salts thereof.

Pharmaceutically acceptable acid addition salts of the compounds of formula I can also be used in the methods and pharmaceutical composition of this invention. Examples of pharmaceutically acceptable acid addition salts of the compounds of formula I are the salts of hydrochloric acid, p-toluenesulfonic acid, fumaric acid, citric acid, succinic acid, salicylic acid, oxalic acid, hydrobromic acid, phosphoric acid, methanesulfonic acid, tartaric acid, maleic acid, di-p-toluoyl tartaric acid, acetic acid, sulfuric acid, hydroiodic acid and mandelic acid.

Unless otherwise indicated, the term "halo", as used herein, includes fluoro, chloro, bromo and iodo.

Unless otherwise indicated, the term "alkyl", as used herein, may be straight, branched or cyclic, and may include straight and cyclic moieties as well as branched and cyclic moieties.

The compounds of formula I may have optical centers and therefore may occur in different enantiomeric configurations. All enantiomers, diastereomers, and other stereoisomers of such compounds of formula I, as well as racemic and other mixtures thereof are included in the pharmaceutical compositions and methods of this invention.

The pharmaceutical compositions and methods of this invention also relates to all radiolabelled forms of the compounds of the formula I. Preferred radiolabelled compounds of formula I are those wherein the radiolabels are selected from ³H, ¹¹C, ¹⁴C, ¹⁸F, ¹²³I and ¹²⁵I. Such radiolabelled compounds are useful as research and diagnostic tools in metabolism pharmacokinetics studies and in binding assays in both animals and man.

Preferred embodiments of formula I include the following compounds of the formula I and their pharmaceutically acceptable salts:
[2-(3,4-Dichlorophenoxy)-5-fluorobenzyl]-dimethylamine;
[2-(3,4-Dichlorophenoxy)-5-fluorobenzyl]-methylamine;
[2-(3,4-Dichlorophenoxy)-5-trifluoromethylbenzyl]-dimethylamine;
N-[4-(3,4-Dichlorophenoxy)-3-dimethylaminomethylphenyl]-acetamide;
1-[2-(3,4-Dichlorophenoxy)phenyl]-ethyl}-dimethylamine;
[2-(3,4-Dichlorophenoxy)-4-trifluoromethylbenzyl]-dimethylamine;
[2-(3,4-Dichlorophenoxy)-4-trifluoromethylbenzyl]-methylamine;
[4-Chloro-2-(3,4-dichlorophenoxy)-benzyl]-methylamine;
{1-[2-(3,4-Dichlorophenoxy)-5-fluorophenyl]-ethyl}-methylamine;
{1-[2-(3,4-Dichlorophenoxy)phenyl}-ethyl}-methylamine;
{1-[2-(4-Chlorophenoxy)phenyl]ethyl}-methylamine;
[2-(3,4-Dichlorophenoxy)-5-methoxybenzyl]-methylamine;
[2-(4-Chlorophenoxy)-5-fluorobenzyl]-methylamine; and
{1-[2-(4-Chlorophenoxy)-5-fluorophenyl]-ethyl}-methylamine.
[2-(3,4-Dichlorophenoxy)-5-methylbenzyl]-dimethylamine;
[4-Bromo-2-(3,4-dichlorophenoxy)-benzyl]-methylamine;
[5-Bromo-2-(3,4-dichlorophenoxy)-benzyl]-methylamine;
[2-(3,4-Dichlorophenoxy)-4,5-dimethoxybenzyl]-methylamine;
[2-(3,4-Dichlorophenoxy)-4-methoxybenzyl]-dimethylamine;
4-(3,4-Dichlorophenoxy)-3-methylaminomethyl-benzonitrile;
[2-(3,4-Dichlorophenoxy)-4,5-dimethylbenzyl]-methylamine;
3-(3,4-Dichlorphenoxy)-4-methylaminomethyl-benzonitrile;
(+)-{1-[2-(3,4-Dichlorophenoxy)-5-fluorophenyl]-ethyl}-methylamine;
(-)-{1-[2-(3,4-Dichlorophenoxy)-5-fluorophenyl]-ethyl}-methylamine;
[2-(3,4-Dichlorophenoxy)-5-trifluoromethyl-benzyl]-methylamine;
[2-(3,4-Dichlorophenoxy)-4-methoxybenzyl]-methylamine;
[2-(4-Chloro-3-fluorophenoxy)-5-fluorobenzyl]-methylamine;
[2-(3-Chloro-4-fluorophenoxy)-5-fluorobenzyl]-methylamine;
(+/-)-2-[2-(3,4-Dichlorophenoxy)-5-fluorophenyl]-pyrrolidine;
(-)-2-[2-(3,4-Dichlorophenoxy)-5-fluorophenyl]-pyrrolidine;
(+)-2-[2-(3,4-Dichlorophenoxy)-5-fluorophenyl]-pyrrolidine; and
2-[2-(3,4-Dichlorophenoxy)-5-fluorophenyl]-N-methylpyrrolidine.

Other embodiments of formula I include the following compounds and their pharmaceutically acceptable salts:
{1-[2-(3,4-Dichlorophenoxy)-5-fluorophenyl]-1-methylethyl}-methylamine;
{1-[2-(3,4-Dichlorophenoxy)-5-fluorophenyl]-1-methylethyl}-dimethylamine;
[4-Chloro-2-(4-chlorophenoxy)-5-fluorobenzyl]-methylamine;
[2-(3,4-Dichlorophenoxy)-5-fluoro-4-methoxybenzyl]-methylamine;
[4-(3,4-Dichlorophenoxy)-3-(dimethylaminomethyl)-phenyl]-dimethylamine
[5-Fluoro-2-(4-fluoro-3-methoxyphenoxy)-benzyl]-dimethylamine;
[2-(4-Chlorophenoxy)-5-isopropylbenzyl]-methylamine;
{1-[2-(4-Chlorophenoxy)-5-trifluoromethylphenyl]-ethyl}-methylamine;
[2-(4-Chlorophenoxy)-4,5-dimethylbenzyl]-methylamine;
{1-[5-Chloro-2(3,4-dichlorophenoxy)phenyl]-propyl}-methylamine;
[2-(3,4-Dichlorophenoxy)-5-methylsulfanyl-benzyl]-methylamine;
{1-[2-(3,4-Dichlorophenoxy)-5-methylsulfanyl-phenyl]-ethyl}-methylamine;
{1-[2-(3,4-Dichloro-phenoxy)-5-methylsulfanyl-phenyl]-1-methylethyl}-methylamine;
[2-(3,4-Dichlorophenoxy)-5-methylsulfanyl-benzyl]-dimethylamine;
[2-(3,4-Dichlorophenoxy)-5-methanesulfinyl-benzyl]-dimethylamine ;
[2-(3,4-Dichlorophenoxy)-5-methanesulfinyl-benzyl]-methylamine;
[2-(3,4-Dichlorophenoxy)-5-methanesulfonyl-benzyl]-methylamine;
[2-(3,4-Dichlorophenoxy)-5-methanesulfonyl-benzyl]-dimethylamine;
[2-(3,4-Dichlorophenoxy)-5-(propane-2-sulfonyl)-benzyl]-methylamine;
2-[2-(3,4-Dichlorophenoxy)-5-fluorophenyl]-piperidine;
2-[2-(3,4-Dichlorophenoxy)-5-fluorophenyl]-1-methyl-piperidine;
3-[2-(3,4-Dichlor-phenoxy)-5-fluorophenyl]-4-methyl-morpholine;
2-[2-(3,4-Dichlorophenoxy)-5-fluorophenyl]-1,2-dimethyl-piperidine;
{1-[2-(3,4-Dichlorophenoxy)-5-fluorophenyl]-cyclopropyl}-dimethylamine;
2-[2-(3,4-Dichlorophenoxy)-5-fluorophenyl]-1,5-dimethyl-pyrrolidine;
3-[2-(3,4-Dichlorophenoxy)-5-fluorophenyl]-4-methyl-thiomorpholine;
{1-[2-(3,4-Dichlorophenoxy)-5-fluorophenyl]-cyclopentyl}-methylamine;
{1-[2-(3,4-Dichlorophenoxy)-5-(propane-2-sulfonyl)-phenyl]-ethyl}-methylamine; and
[4-Chloro-2-(3,4-dichlorophenoxy)-5-methanesulfonyl-benzyl]-methylamine.

Other embodiments of this invention relate to the compound of the formula I wherein m is zero, n is one, R³ and R⁴ are hydrogen, X is chloro, bromo, iodo or methyl, R¹ is hydrogen and R² is methyl.

Other examples of Serotonin Reuptake Inhibitors (SRI) that can be used in the method and pharmaceutical compositions of this invention are compounds of the formula: 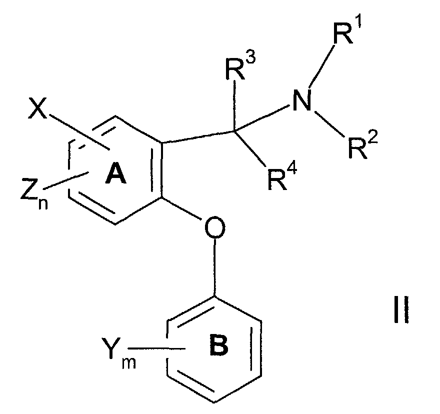 wherein
phenyl ring A and phenyl ring B can each, independently, be replaced by a naphthyl group, and wherein when phenyl ring A is replaced by a naphthyl group, the ethereal oxygen of Formula II and the carbon to which R³, R⁴ and NR¹R² are attached, are attached to adjacent ring carbon atoms of the naphthyl group and neither of said adjacent ring carbon atoms is also adjacent to a fused ring carbon atom of said naphthyl group;
n and m are selected, independently, from one, two and three;
R¹ and R² are selected, independently, from hydrogen, (C₁-C₄)alkyl, (C₂-C₄)alkenyl, and (C₂-C₄)alkynyl, or R¹ and R², together with the nitrogen to which they are attached, form a four to eight membered saturated ring containing one or two heteroatoms, including the nitrogen to which R¹ and R² are attached, wherein the second heteroatom, when present, is selected from oxygen, nitrogen and sulfur, and wherein said ring may optionally be substituted at available binding sites with from one to three substituents selected, independently, from hydroxy and (C₁-C₆)alkyl;
R³ and R⁴ are selected, independently, from hydrogen and (C₁-C₄) alkyl optionally substituted with from one to three fluorine atoms, or R³ and R⁴ together with the carbon to which they are attached form a four to eight membered saturated carbocyclic ring, and wherein said ring may optionally be substituted at available binding sites with from one to three substituents selected, independently, from hydroxy and (C₁-C₆)alkyl;
or R² and R³, together with the nitrogen to which R² is attached and the carbon to which R³ is attached, form a four to eight membered saturated ring containing one or two heteroatoms, including the nitrogen to which R² is attached, wherein the second heteroatom, when present, is selected from oxygen, nitrogen and sulfur, and wherein said ring may optionally be substituted at available binding sites with from one to three substituents selected, independently, from hydroxy and (C₁-C₆)alkyl;
each X is selected, independently, from phenyl, heteroaryl (e.g., furan, thiophene, pyrrole, thiazole, isothiazole, oxazole, isoxazole, imidazole, 1,2,4-oxadiazole, 1,2,4-thiadiazole, 1,2,4-triazole, 1,2,3,-triazole, tetrazole, pyridine, pyrimidine, pyrazine, quinoline, isoquinoline, quinazoline, quinoxaline, benzothiophene, benzofuran, benzimidazole, benzisoxazole, benzisothiazole and indole) or heterocycle (e.g., imidazolidine, oxazolidine, thiazolidine, pyrrolidine, piperidine, morpholine) groups as defined below and may be further substituted by hydrogen, halo (i.e., fluorine, chlorine, bromine, iodine), (C₁-C₄)alkyl optionally substituted with from one to three fluorine atoms, (C₁-C₄)alkoxy optionally substituted with from one to three fluorine atoms, cyano, nitro, amino, hydroxy, carbonyl, (C₁-C₄)alkylamino, di-[(C₁-C₄)alkyl]amino, NR⁵(C=O)(C₁-C₄)alkyl, SO₂NR⁵R⁶ and SOₚ(C₁-C₆)alkyl, wherein R⁵ and R⁶ are selected, independently, from hydrogen and (C₁-C₆)alkyl, and p is zero, one or two;
each Y is selected, independently, from hydrogen, halo (i.e., chloro, fluoro, bromo or iodo), (C₁-C₄)alkyl optionally substituted with from one to three fluorine atoms, (C₁-C₄)alkoxy optionally substituted with from one to three fluorine atoms, cyano, nitro, amino, (C₁-C₄)alkylamino, di-[(C₁-C₄)alkyl]amino, NR⁵(C=O)(C₁-C₄)alkyl, SO₂NR⁵R⁶ and SOₚ(C₁-C₆)alkyl, wherein R⁵ and R⁶ are selected, independently, from hydrogen and (C₁-C₆)alkyl, and p is zero, one or two; and
each Z is selected independently from hydrogen, halo (i.e., chloro, fluoro, bromo or iodo), (C₁-C₄)alkyl optionally substituted with from one to three fluorine atoms, (C₁-C₄)alkoxy;
and the pharmaceutically acceptable salts thereof. Compounds of formula II, and their pharmaceutically acceptable salts, have activity in inhibiting reuptake of serotonin, dopamine, and norepinephrine.

In one embodiment, ring B is phenyl, not replaced with a naphthyl group. In another embodiment, phenyl ring B in the compounds of formula II is replaced with a naphthyl group.

In a preferred embodiment when ring B is phenyl, each Y is hydrogen or halo. In a more preferred embodiment, m is 1 or 2, and each Y is chlorine.

In another embodiment, compounds of formula II, or pharmaceutically acceptable salts, thereof are described above, but wherein X is selected from furan, thiophene, pyrrole, and 1,2,3-triazole, and wherein X may be further substituted.

In another embodiment, compounds of formula II or salts thereof are described above, but wherein each Z is selected from hydrogen and halo. Preferably, Z is hydrogen.

In a further embodiment, compounds of formula II or salts thereof are described above, wherein R³ and R⁴ are independently selected from hydrogen and unsubstituted (C₁-C₄) alkyl. Preferably, one or both of R³ and R⁴ are hydrogen.

In a further embodiment, formula II or salts thereof, wherein R¹ and R² are independently selected from hydrogen and unsubstituted (C₁-C₄)alkyl. Preferably, one of R¹ and R² is hydrogen and the other of R¹ and R² is (C₁-C₄)alkyl. More preferably, one of R¹ and R² is hydrogen and the other of R¹ and R² is methyl.

The methods and pharmaceutical compositions of this invention also relates to the pharmaceutically acceptable acid addition salts of the compounds of formula II. Examples of pharmaceutically acceptable acid addition salts of the compounds of formula II are the salts of hydrochloric acid, p-toluenesulfonic acid, fumaric acid, citric acid, succinic acid, salicylic acid, oxalic acid, hydrobromic acid, phosphoric acid, methanesulfonic acid, tartaric acid, maleic acid, di-p-toluoyl tartaric acid, acetic acid, sulfuric acid, hydroiodic acid and mandelic acid.

Unless otherwise indicated, the term "halo", as used herein, includes fluoro, chloro, bromo and iodo.

Unless otherwise indicated, the term "alkyl", as used herein, may be straight, branched or cyclic, and may include straight and cyclic moieties as well as branched and cyclic moieties.

When reference is made to SOₚ(C₁-C₆)alkyl, and p is two, this indicates a sulfone, in other words, S(=O)₂(C₁-C₆)alkyl.

The compounds of formula II may have optical centers and therefore may occur in different enantiomeric configurations. The invention includes all enantiomers, diastereomers, and other stereoisomers of such compounds of formula II, as well as racemic and other mixtures thereof.

Formula II compounds also include isotopically-labeled compounds, which are identical to those recited in formula II, but for the fact that one or more atoms are replaced by an atom having an atomic mass or mass number different from the atomic mass or mass number usually found in nature. Examples of isotopes that can be incorporated into compounds of the invention include isotopes of hydrogen, carbon, nitrogen, oxygen, phosphorous, fluorine, iodine, and chlorine, such as ³H, ¹¹C, ¹⁴C, ¹⁸F, ¹²³I and ¹²⁵I. Compounds of the present invention and pharmaceutically acceptable salts of said compounds that contain the aforementioned isotopes and/or other isotopes of other atoms are within the scope of this invention. Isotopically-labeled compounds of the present invention, for example those into which radioactive isotopes such as ³H and ¹⁴C are incorporated, are useful in drug and/or substrate tissue distribution assays. Tritiated, i.e., ³H, and carbon-14, i.e., ¹⁴C, isotopes are particularly preferred for their ease of preparation and detectability. Further, substitution with heavier isotopes such as deuterium, i.e., ²H, can afford certain therapeutic advantages resulting from greater metabolic stability, for example increased *in vivo* half-life or reduced dosage requirements and, hence, may be preferred in some circumstances.

Preferred embodiments of the compounds of formula II include the following compounds of the formula II and their pharmaceutically acceptable salts:
[4-(3,4-Dichlorophenoxy)-biphenyl-3-ylmethyl]-methylamine;
[2-(3,4-Dichlorophenoxy)-5-thiophen-3-ylbenzyl]-methylamine;
[2-(3,4-Dichlorophenoxy)-4-thiophen-3-ylbenzyl]-methylamine;
[2-(3,4-Dichlorophenoxy)-4-furan-2-ylbenzyl]-methylamine;
[2-(3,4-Dichlorophenoxy)-5-furan-2-ylbenzyl]-methylamine;
N-[4'-(3,4-Dichlorphenoxy)-3'-methylaminomethyl-biphenyl-3-yl]-acetamide;
[2-(3,4-Dichlorophenoxy)-5-thiophen-2-ylbenzyl]-methylamine;
[4-(3,4-Dichlorophenoxy)-4'-fluoro-biphenyl-3-ylmethyl]-methyamine;
[2-(3,4-Dichlorophenoxy)-5-[1,2,3]triazol-1-ylbenzyl]-methylamine;
[2-(3,4-Dichlorophenoxy)-5-[1,2,3]triazol-2-ylbenzyl]-methylamine;
[2-(3,4-Dichlorophenoxy)-5-pyridin-2-ylbenzyl]-methylamine;
[2-(3,4-Dichlorophenoxy)-5-pyridin-3-ylbenzyl]-methylamine;
1-[4-(3,4-Dichlorophenoxy)-3-methylaminomethylphenyl]-1H-pyrazol-3-ylamine;
[2-(3,4-Dichlorophenoxy)-5-pyridin-4-ylbenzyl]-methylamine;
[3-(3,4-Dichlorophenoxy)-biphenyl-4-ylmethyl]-methylamine;
[4-(3,4-Dichlorophenoxy)-4'-methyl-biphenyl-3-ylmethyl]-methylamine;
[2-(3,4-Dichlorophenoxy)-4-thiophen-2-ylbenzyl]-methylamine;
[2-(3,4-Dichlorophenoxy)-5-pyrimidin-2-ylbenzyl]-methylamine;
[2-(3,4-Dichlorophenoxy)-5-pyrimidin-4-ylbenzyl]-methylamine;
[2-(3,4-Dichlorophenoxy)-5-(2-methylpyrimidin-4-yl)-benzyl]-methylamine;
{1-[2-(3,4-Dichlorophenoxy)-5-(2-methylpyrimidin-4-yl)-phenyl]-ethyl}-methylamine;
4-[4-(3,4-Dichlorophenoxy)-3-(1-methylpyrrolidin-2-yl)-phenyl]-2-methylpyrimidine;
[2-(4-Chlorophenoxy)-5-(1-methyl-1H-pyrrol-3-yl)-benzyl]-dimethylamine;
[5-(1-methyl-1H-pyrrol-3-yl)-2-(naphthalen-2-yloxy)-benzyl]-dimethyl amine;
[5-lmidazol-1-yl-2-(naphthalen-2-yloxy)-benzyl]-dimethylamine;
1,5,5-Trimethyl-3-[3-methylaminomethyl-4-(naphthalen-2-yloxy)-phenyl]-imidazolidine-2,4-dione;
1-Methyl-3-[3-methylaminomethyl-4-(naphthalen-2-yloxy)-phenyl]-imidazolidine-2,4-dione;
3-[3-Methylaminomethyl-4-(naphthalen-2-yloxy)-phenyl]-thiazolidine-2,4-dione;
3-[3-Methylaminomethyl-4-(naphthalen-2-yloxy)-phenyl]-oxazolidine-2,4-dione;
3-[3-Methylaminomethyl-4-(naphthalen-2-yloxy)-phenyl]-oxazolidin-2-one;
3-[3-Methylaminomethyl-4-(naphthalen-2-yloxy)-phenyl]-thiazolidin-2-one;
1-Methyl-3-[3-methylaminomethyl-4-(naphthalen-2-yloxy)-phenyl]-imidazolidin-2-one;
1-Methyl-3-[3-methylaminomethyl-4-(naphthalen-2-yloxy)-phenyl]-tetrahydropyrimidin-2-one;
1-[4-(3,4-Dichlorophenoxy)-3-methylaminomethyl-phenyl]-3-methyltetrahydropyrimidin-2-one;
1-[4-(3,4-Dichlorophenoxy)-3-methylaminomethyl-phenyl]-3-methylimidazolidin-2-one;
3-[4-(3,4-Dichlorophenoxy)-3-methylaminomethyl-phenyl]-thiazolidin-2-one;
3-[4-(3,4-Dichlorophenoxy)-3-methylaminomethyl-phenyl]-oxazolidin-2-one;
[2-(3,4-Dichlorophenoxy)-5-(2-methylthiazol-4-yl)-benzyl]-methylamine;
[2-(3,4-Dichlorophenoxy)-5-(2-methyloxazol-4-yl)-benzyl]-methylamine;
[2-(3,4-Dichlorophenoxy)-5-(2,5-dimethyloxazol-4-yl)-benzyl]-methylamine;
[2-(3,4-Dichlorophenoxy)-5-(2,5-dimethylthiazol-4-yl)-benzyl]-methylamine;
[2-(3,4-Dichlorophenoxy)-5-(5-methyl-[1,2,4]thiadiazol-3-yl)-benzyl]-methylamine;
[2-(3,4-Dichlorophenoxy)-5-(5-methyl-[1,2,4]oxadiazol-3-yl)-benzyl]-methylamine;
[2-(3,4-Dichlorophenoxy)-5-[1,2,3]oxadiazol-4-yl-benzyl]-methylamine;
[2-(3,4-Dichlorophenoxy)-5-(5-methyl-[1,2,3]thiadiazol-4-yl)-benzyl]-methylamine;
[2-(3,4-Dichlorophenoxy)-5-(2,4-dimethyloxazol-5-yl)-benzyl]-methylamine;
[2-(3,4-Dichlorophenoxy)-5-(2,4-dimethylthiazol-5-yl)-benzyl]-methylamine;
[2-(3,4-Dichlorophenoxy)-5-[1,2,4]triazol-1-ylbenzyl]-methylamine;
[2-(3,4-Dichlorophenoxy)-5-(3-methyl-[1,2,4]triazol-1-yl)-benzyl]-methylamine;
[2-(4-Chlorophenoxy)-5-(3,5-dimethyl-[1,2,4]triazol-1-yl)-benzyl]-methylamine;
[2-(4-Chlorophenoxy)-5-tetrazol-1-ylbenzyl]-methylamine;
[2-(4-Chlorophenoxy)-5-(5-methyltetrazol-1-yl)-benzyl]-dimethylamine;
[2-(4-Chlorophenoxy)-5-[1,2,4]triazol-4-ylbenzyl]-dimethylamine;
[2-(4-Chlorophenoxy)-5-(1-methyl-1H-tetrazol-5-yl)-benzyl]-dimethylamine; and
{1-[2-(3,4-Dichlorophenoxy)-5-(1-methyl-1H-tetrazol-5-yl )-phenyl]-ethyl}-dimethylamine.

Suitable classes of a neurotransmitter-inducing or precursor agent that may be used in the compositions and methods of this invention include, but not limited to the following compounds:
L-phenylalanine;
L-tryptophan;
L-tyrosine; L-DOPA or tyramine

### Detailed Description Of The Invention

The following references refer to novel biaryl ether derivatives useful as monoamine reuptake inhibitors that exhibit activity as Serotonin Reuptake Inhibitor and that can be used, in combination with neurotransmitter-inducing or precursor agent in the pharmaceutical compositions and methods of this invention, and to methods of preparing the same: PCT application No.: PCT/IB00/01373 Filed Sept 27, 2000 and PCT application No. PCT/IB00/00108 filed Feb 2, 2000. United States Patent No. 4,018,830, issued April 19, 1997, refers to phenylthioaralkylamines and 2-phenylthiobenzylamines which are active as antiarrhythmics.

WO 97/17325, International Publication Date May 15, 1997, refers to derivatives of N,N-dimethyl-2-(arylthio)benzylamine which selectively influence serotonin transport in the central nervous system and are useful as antidepressants.

United States Patent 5,190,965, issued March 2, 1993, and United States Patent 5,430,063, issued July 4, 1995, refer to phenoxyphenyl derivatives which have utility in the treatment of depression.

United States Patent 4,161,529, issued July 17, 1979, refers to pyrrolidine derivatives that possess anticholesteremic and hypolipemic activity.

United States Provisional Application No. 60/121313, filed February 23, 1999, refers to biaryl ethers that have activity in inhibiting reuptake of both serotonin and dopamine. The foregoing patents and patent applications are incorporated herein by reference in their entirety.

The (SRI) antidepressants and anxiolytics of the formula I can be prepared as described in the following patent application, which is referred to above and incorporated herein by reference in its entirety; PCT application NO. PCT/IB00/01373 filed September 27, 2000. (SRI) antidepressants and anxiolytic of Formula II and III can be prepared as described in the following patent application, which is referred to above and incorporated herein by reference in its entirety: PCT application No. PCT/IB00/00108 filed February 2, 2000.

All the foregoing patents and patent applications are incorporated herein by reference in their entirety.

A neurotransmitter-inducing or precursor agent is a component which enhances or triggers production of a neurotransmitter.

A preferred neurotransmitter-inducing or precursor agent for use in the present invention is L-phenylalanine (LPA). However L-tryptophan, L-tyrosine, L-Dopa, and tyramine are also useful in the present invention.

Compounds may be provided as a metabolite of a precursor. For example, L-phenylalanine may be provided as a metabolite of aspartame. L-DOPA can also be used as a metabolite of aspartame.

This invention relates both to methods of treating Multiple Sclerosis, other demyelinating conditions and peripheral neuropathy in which the neurotransmitter-inducing or precursor agent and the (SRI) anxiolytic or antidepressant agent, or pharmaceutically acceptable salts of the same, are administered together, as part of the same pharmaceutical composition, as well as to methods in which these two active agents are administered separately as part of an appropriate dose regimen designed to obtain the benefits of the combination therapy. The appropriate dose regimen, the amount of each dose administered, and specific intervals between doses of each active agent will depend upon the subject being treated, and the severity of the condition. Generally, in carrying out the methods of this invention, the neurotransmitter-inducing or precursor agent will be administered to an adult human in an amount ranging from about 100 mg to 5 g per day in single or divided doses, preferably from about 500-2000 mg per day. The compounds may be administered on a regimen of up to 6 times per day, preferably 1 to 4 times per day, especially 2 times per day and most especially once daily. A suitable dosage level for the (SRI) antidepressant agent is about 0.5 to 1500 mg per day, preferably about 2.5 to 1000 mg per day, and especially about 2.5 to 500 mg per day. The compounds may be administered on a regimen of up to 6 times per day, preferably 1 to 4 times per day, especially 2 times per day and most especially once daily. Variations may nevertheless occur depending upon the species of animal being treated and its individual response to said medicament, as well as on the type of pharmaceutical formulation chosen and the time period and interval at which such administration is carried out. In some instances, dosage levels below the lower limit of the aforesaid range may be more than adequate, while in other cases still larger doses may be employed without causing any harmful side effect, provided that such larger doses are first divided into several small doses for administration throughout the day.

The neurotransmitter-inducing or precursor agents and their pharmaceutically acceptable salts, and the (SRI) antidepressant and anxiolytic agents and their pharmaceutically acceptable salts that are employed in the pharmaceutical compositions and methods of this invention are hereinafter also referred to as "therapeutic agents". The therapeutic agents can be administered via either the oral or parenteral route. Compositions containing both a neurotransmitter-inducing or precursor agent and an (SRI) anxiolytic or antidepressant agent, or pharmaceutically acceptable salts of one or both therapeutic agents, will generally be administered orally or parenterally daily, in single or divided doses, so that the total amount of each active agent administered falls within the above guidelines.

The therapeutic agents may be administered alone or in combination with pharmaceutically acceptable carriers or diluents by either of the routes previously indicated, and such administration may be carried out in single or multiple doses. More particularly, the therapeutic agents of this invention can be administered in a wide variety of different dosage forms, *i*.*e*., they may be combined with various pharmaceutically acceptable inert carriers in the form of tablets, capsules, lozenges, troches, hard candies, suppositories, aqueous suspensions, injectable solutions, elixirs, syrups, and the like. Such carriers include solid diluents or fillers, sterile aqueous media and various non-toxic organic solvents, etc. Moreover, oral pharmaceutical compositions can be suitably sweetened and/or flavored. In general, the therapeutic agents of this invention, when administered separately (*i*.*e*., not in the same pharmaceutical composition) are present in such dosage forms at concentration levels ranging from about 5.0% to about 70% by weight.

For oral administration, tablets containing various excipients such as microcrystalline cellulose, sodium citrate, calcium carbonate, dicalcium phosphate and glycine may be employed along with various disintegrants such as starch (and preferably corn, potato or tapioca starch), alginic acid and certain complex silicates, together with granulation binders like polyvinylpyrrolidone, sucrose, gelatin and acacia. Additionally, lubricating agents such as magnesium stearate, sodium lauryl sulfate and talc are often very useful for tabletting purposes. Solid compositions of a similar type may also be employed as fillers in gelatin capsules; preferred materials in this connection also include lactose or milk sugar as well as high molecular weight polyethylene glycols. When aqueous suspensions and/or elixirs are desired for oral administration, the active ingredient may be combined with various sweetening or flavoring agents, coloring matter or dyes, and, if so desired, emulsifying and/or suspending agents as well, together with such diluents as water, ethanol, propylene glycol, glycerin and various like combinations thereof.

For parenteral administration, solutions of a therapeutic agent in either sesame or peanut oil or in aqueous propylene glycol may be employed. The aqueous solutions should be suitably buffered if necessary and the liquid diluent first rendered isotonic. These aqueous solutions are suitable for intravenous injection purposes. The oily solutions are suitable for intraarticular, intramuscular and subcutaneous injection purposes. The preparation of all these solutions under sterile conditions is readily accomplished by standard pharmaceutical techniques well known to those skilled in the art.

As stated above, the neurotransmitter-inducing or precursor agent and the (SRI) anxiolytic or antidepressant agent may be formulated in a single pharmaceutical composition or alternatively in individual pharmaceutical compositions for simultaneous, separate or sequential use in accordance with the present invention.

Preferably the compositions according to the present invention, which contain both a neurotransmitter-inducing or precursor agent and an (SRI) anxiolytic agent or an antidepressant, as well as the pharmaceutical compositions used to deliver only one of these active agents, are in unit dosage forms such as tablets, pills, capsules, powders, granules, solutions or suspensions, or suppositories, for oral, parenteral or rectal administration, by inhalation or insufflation or administration by transdermal patches or by buccal cavity absorption wafers.

For preparing solid compositions such as tablets, the principal active ingredient is mixed with a pharmaceutical carrier, *e.g.*, conventional tableting ingredients such as corn starch, lactose, sucrose, sorbitol, talc, stearic acid, magnesium stearate, dicalcium phosphate or gums, and other pharmaceutical diluents, *e.g.*, water, to form a solid preformulation composition containing a homogeneous mixture of a compound of the present invention, or a non-toxic pharmaceutically acceptable salt thereof. When referring to these preformulation compositions as homogeneous, it is meant that the active ingredient is dispersed evenly throughout the composition so that the composition may be readily subdivided into equally effective unit dosage forms such as tablets, pills and capsules. This solid preformulation composition is then subdivided into unit dosage forms of the type described above containing, typically, from 0.05 to about 500 mg of each of the therapeutic agents contained in the composition. The tablets or pills of the composition can be coated or otherwise compounded to provide a dosage form affording the advantage of prolonged action. For example, the tablet or pill can comprise an inner dosage and an outer dosage component, the latter being in the form of an envelope over the former. The two components can be separated by an enteric layer which serves to resist disintegration in the stomach and permits the inner component to pass intact into the duodenum or to be delayed in release. A variety of materials can be used for such enteric layers or coatings, such materials including a number of polymeric acids and mixtures of polymeric acids with such materials as shellac acetyl alcohol and cellulose acetate.

The liquid forms in which the novel compositions of the present invention may be incorporated for administration orally or by injection include aqueous solutions, suitably flavored syrups, aqueous or oil suspensions, and flavored emulsions with edible oils such as cottonseed oil, sesame oil, coconut oil, peanut oil or soybean oil, as well as elixirs and similar pharmaceutical vehicles. Suitable dispersing or suspending agents for aqueous suspensions include synthetic and natural gums such as tragacanth, acacia, alginate, dextran, sodium carboxymethyl cellulose, methylcellulose, polyvinyl-pyrrolidone or gelatin.

Preferred compositions for administration of therapeutic agent or other therapeutic agent by injection include those comprising the therapeutic agent in association with a surface-active agent (or wetting agent or surfactant) or in the form of an emulsion (as a water-in-oil or oil-in-water emulsion).

Suitable surface-active agents include, in particular, non-ionic agents, such as polyoxyethylenesorbitans (*e.g.*, Tween™ 20, 40, 60, 80 or 85) and other sorbitans (*e.g.*, Span™ 20, 40, 60, 80 or 85). Compositions with a surface-active agent will conveniently comprise between 0.05 and 5% surface-active agent, and preferably between 0.1 and 2.5%. It will be appreciated that other ingredients may be added, for example mannitol or other pharmaceutically acceptable vehicles, if necessary.

Suitable emulsions may be prepared using commercially available fat emulsions, such as Intralipid™, Liposyn ™, Infonutrol™, Lipofundin ™ and Lipiphysan™. The therapeutic agent may be either dissolved in a pre-mixed emulsion composition or alternatively it may be dissolved in an oil (*e.g.*, soybean oil, safflower oil, cottonseed oil, sesame oil, com oil or almond oil) and an emulsion formed upon mixing with a phospholipid (*e.g.*, eggs phospholipids, soybean phospholipids or soybean lecithin) and water. It will be appreciated that other ingredients may be added, for example glycerol or glucose, to adjust the tonicity of the emulsion. Suitable emulsions will typically contain up to 20% oil, for example, between 5 and 20%. The fat emulsion will preferably comprise fat droplets between 0.1 and 1.0 µm, particularly 0.1 and 0.5 µm, and have a pH in the range of 5.5 to 8.0.

Compositions for inhalation or insufflation include solutions and suspensions in pharmaceutically acceptable, aqueous or organic solvents or mixtures thereof, and powders. The liquid or solid compositions may contain suitable pharmaceutically acceptable excipients as set out above. Preferably the compositions are administered by the oral or nasal respiratory route for local or systemic effect. Compositions in preferably sterile pharmaceutically acceptable solvents may be nebulised by use of inert gases. Nebulised solutions may be breathed directly from the nebulising device or the nebulising devise may be attached to a face mask, tent or intermittent positive pressure breathing machine. Solution, suspension, or powder compositions may be administered, preferably orally or nasally, from devices which deliver the formulation in an appropriate manner.

Compositions of the present invention may also be presented for administration in the form of transdermal patches using conventional technology. The compositions may also be administered via the buccal cavity using, for example, absorption wafers.

The present invention further provides a process for the preparation of a pharmaceutical composition comprising a neurotransmitter-inducing or precursor agent and an (SRI) antidepressant or anxiolytic agent, or pharmaceutically acceptable salts of the same, which process comprises bringing a neurotransmitter-inducing or precursor agent and the (SRI) antidepressant or anxiolytic agent (or the pharmaceutically acceptable salts of one or both of these therapeutic agents) into association with a pharmaceutically acceptable carrier or excipient.

It will be appreciated that the amount of the neurotransmitter-inducing or precursor agent and the (SRI) antidepressant or anxiolytic agent required for use in the treatment of MS, other demyelinating conditions and peripheral neuropathy will vary not only with the particular compounds or compositions selected but also with the route of administration, the nature of the condition being treated, and the age and condition of the patient, and will ultimately be at the discretion of the patient's physician or pharmacist.

The *in vitro* activity of the (SRI) compounds used in this invention at the individual monoamine reuptake sites can be determined using rat synaptosomes or HEK-293 cells transfected with the human serotonin, dopamine or norepinephrine transporter, according to the following procedure adapted from those described by S. Snyder et al., (Molecular Pharmacology, **1971,** 7, 66-80), D.T. Wong et al., (Biochemical Pharmacology, **1973,** 22, 311-322), H. F. Bradford (Journal of Neurochemistry, **1969,** 16, 675-684) and D. J. K. Balfour (European Journal of Pharmacology, **1973,** 23, 19-26).

Synaptosomes: Male Sprague Dawley rats are decapitated and the brains rapidly removed. The cortex, hippocampi and corpus striata are dissected out and placed in ice cold sucrose buffer, 1 gram in 20 ml of buffer (the buffer is prepared using 320 mM sucrose containing 1mg/ml glucose, 0.1mM ethylenediamine tetraacetic acid (EDTA) adjusted to pH 7.4 with tris(hydroxymethyl)-aminomethane (TRIS) base). The tissues are homogenized in a glass homogenizing tube with a Teflon™ pestle at 350 rpm using a Potters homogenizer. The homogenate is centrifuged at 1000 x g for 10 min. at 4°C. The resulting supernatant is recentrifuged at 17,000 x g for 20 min. at 4°C. The final pellet is resuspended in an appropriate volume of sucrose buffer that yielded less than 10% uptake.

Cell Preparation: HEK-293 cells transfected with the human serotonin (5-HT), norepinephrine (NE) or dopamine (DA) transporter are grown in DMEM (Dulbecco's Modified Eagle Medium, Life Technologies Inc., 9800 Medical Center Dr., Gaithersburg, MD, catalog no. 11995-065)) supplemented with 10% dialyzed FBS (Fetal Bovine Serum, from Life Technologies, catalog no. 26300-053), 2 mM L-glutamine and 250 ug/ml G418 for the 5-HT and NE transporter or 2ug/ml puromycin for the DA transporter, for selection pressure. The cells are grown in Gibco triple flasks, harvested with Phosphate Buffered Saline (Life Technologies, catalog no. 14190-136) and diluted to an appropriate amount to yield less than 10% uptake.

Neurotransmitter Uptake Assay: The uptake assays are conducted in glass tubes containing 50 uL of solvent, inhibitor or 10uM sertraline, desipramine or nomifensine for the 5-HT, NE or DA assay nonspecific uptake, respectively. Each tube contains 400 uL of [3H]5-HT (5 nM final), [3H]NE (10 nM final) or [3H]DA (5 nM final) made up in modified Krebs solution containing 100 uM pargyline and glucose (1mg/ml). The tubes are placed on ice and 50 uL of synaptosomes or cells is added to each tube. The tubes are then incubated at 37° C for 7 min. (5-HT, DA) or 10 min. (NE). The incubation is terminated by filtration (GF/B filters), using a 96-well Brandel Cell Harvester, the filters are washed with modified Krebs buffer and counted using either a Wallac Model 1214 or Wallac Beta Plate Model 1205 scintillation counter.

Determination of the *in vivo* serotonin reuptake inhibition activity and potency of action for the compounds of the present invention can be made by measuring the ability of the compound to block the depletion of serotonin in the anterior cortex induced by (+/-)-parachloroamphetamine (PCA) in the rat, according to a procedure adapted from R. W. Fuller, H. D. Snoddy and M. L. Cohen in Neuropharmacology 23: 539-544 (1984).

Generally, male, white Sprague-Dawley rats weighing 160-230 g each are assigned to either the control (vehicle) or test groups. When the test compound is administered subcutaneously (sc) at a given dose, it is co-administered with 5 mg/kg of parachloroamphetamine (PCA). Three hours post-dose, the animals are sacrificed by decapitation and the anterior cortices are removed, wrapped in parafilm and frozen in dry ice (-78 C). When dosed orally (po), the rats are fasted the night before the experiment and then treated with the test compound at a given dose 30 minutes prior to the administration of the PCA (5 mg/kg, sc). After three hours, the animals are sacrificed and the tissues removed as above.

To determine the serotonin (5-HT) levels, the frozen tissues are homogenized with Branson sonifier in 0.5 mL of mobile phase in Eppendorf centrifuge tubes. Samples are then spun down at 11000 rpm for twenty minutes in a Sorval SH-MT rotor in a Sorval RC5C centrifuge. The supernatant thus obtained is pipetted into HPLC vials and the 5-HT levels are measured on HPLC-EC.

Interpretation of the results is as follows: Each experiment has a set of vehicle treated animals and a set of PCA-only animals. The mean 5-HT value of the PCA animals is subtracted from the mean 5-HT value of the vehicle animals. This is the signal or window of the response. The mean 5-HT value of each test group is determined, the mean of the PCA group subtracted from that, and that amount divided by the window is the per cent protection from the PCA effect for that dose. To report an ID₅₀, a line is drawn mathematically through the per cent protection values and the 50 per cent level calculated.

All of the title compounds of Formula I and II and III were assayed *in vitro* for serotonin, dopamine, and norepinephrine reuptake inhibition, and all had IC₅₀ values of about less than or equal to 250 nM for serotonin reuptake inhibition, about less than or equal to 1000 nM for dopamine reuptake inhibition, and about less than or equal to 1000 nM for norepinephrine reuptake inhibition.

The diabetic neuropathy with which the present invention is concerned may be characterized by degeneration of the long nerves (the nerves of the peripheral nervous system) as a result of the metabolic disturbances of diabetes. This can be contrasted with other neurodegenerative disorders such Multiple Sclerosis, the effects of which are concentrated in the central nervous system. While multiple sclerosis leads to demyelination of the myelin sheath (which surrounds the neurons), the toxic effects of diabetes occur in the body of the peripheral neuron, possible due to the toxic effect of metabolites arising through the underlying diabetic disturbance of carbohydrate metabolism, or as a secondary effect of diabetic microvascular degeneration. Whatever the mechanism, the result of the degenerative changes in the body of the peripheral neuron is reduced signal conductivity along the length of the nerve. It is believed that the initial generation of a signal and the passage of a signal across synapses may not be directly effected by the condition.

In addition to diabetic neuropathies, the present invention is applicable to any and all of peripheral neuropathies, particularly painful neuropathies, including those listed above.

A neurotransmitter inducing or precursing agent is a component which enhances or triggers production of a neurotransmitter.

A preferred neurotransmitter-inducing or precursing agent for use in the present invention is L-phenylalanine (LPA). However L-tryptophan may also find use in the present invention.

Other amino acids such as L-tyrosine or other compounds such as tyramine may also fine use in the present invention as a neurotransmitter, inducer or precursor.

Compounds may be provided as a metabolite of a precursor. For example, L-phenylalanine may be provided as a metabolite of aspartame.

When administered in combination, either as a single or as separate pharmaceutical composition(s), the neurotransmitter-inducing or precursor agent and an (SRI) antidepressant or anti-anxiety agent, are presented in a ratio which is consistent with the manifestation of the desired effect. In particular, the ratio by weight of the neurotransmitter-inducing or precursor agent and the (SRI) antidepressant or anxiolytic agent will suitably be between 0.001 to 1 and 1000 to 1, and especially between 0.01 to I and 100 to 1.

As used herein the term "mammal" includes animals of economic importance such as bovine, ovine, and porcine animals, especially those that produce meat, as well as domestic animals (*e.g.* cats and dogs), sports animals (*e.g.* horses), zoo animals, and humans, the latter being preferred.

## Claims

1. A combination treatment comprising: (a) a compound that exhibits activity, respectively, as an (SRI) anxiolytic agent or an antidepressant, or a pharmaceutically acceptable salt thereof; and (b) a neurotransmitter-inducing or precursor agent or pharmaceutically acceptable salt thereof.

2. A combination according to claim 2 suitable for the simultaneous, sequential or separate treatment of Multiple Sclerosis and other demyelinating conditions in a mammal and wherein the active agents "a" and "b" are present in amounts that render the composition effective in treating, respectively, Multiple Sclerosis and other demyelinating conditions.

3. A combination according to any one of claims 1-2, wherein the (SRI) anxiolytic agent or antidepressant is selected from compounds of the formula I: 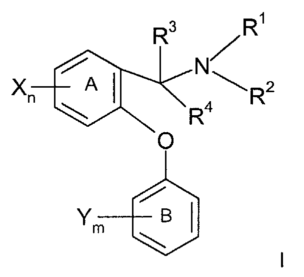 wherein
phenyl ring A and phenyl ring B can each, independently, be replaced by a naphthyl group, and wherein when phenyl ring A is replaced by a naphthyl group, the ethereal oxygen of structure I and the carbon to which R³, R⁴ and NR¹R² are attached, are attached to adjacent ring carbon atoms of the naphthyl group and neither of said adjacent ring carbon atoms is also adjacent to a fused ring carbon atom of said naphthyl group;
n and m are, selected, independently, from one, two and three;
R¹ and R² are selected, independently, from hydrogen (C₁-C₄)alkyl, (C₂-C₄)alkenyl, and (C₂-C₄)alkynyl, or R¹ and R², together with the nitrogen to which they are attached, form a four to eight membered saturated ring containing one or two heteroatoms, including the nitrogen to which R¹ and R² are attached, wherein the second heteroatom, when present, is selected from oxygen, nitrogen and sulfur, and wherein said ring may optionally be substituted at available binding sites with from one to three substituents selected, independently, from hydroxy and (C₁-C₆)alkyl;
R³ and R⁴ are selected, independently, from hydrogen and (C₁-C₄) alkyl optionally substituted with from one to three fluorine atoms, or R³ and R⁴, together with the carbon to which they are attached, form a four to eight membered saturated carbocyclic ring, and wherein said ring may optionally be substituted at available binding sites with from one to three substituents selected, independently, from hydroxy and (C₁-C₆)alkyl;
or R² and R³, together with the nitrogen to which R² is attached and the carbon to which R³ is attached, form a four to eight membered saturated ring containing one or two heteroatoms, including the nitrogen to which R² is attached, wherein the second heteroatom, when present, is selected from oxygen, nitrogen and sulfur, and wherein said ring may optionally be substituted at available binding sites with from one to three substituents selected, independently, from hydroxy and (C₁-C₆)alkyl;
each X and each Y is selected, independently, from hydrogen, halo (i.e., chloro, fluoro, bromo or iodo), (C₁-C₄)alkyl optionally substituted with from one to three fluorine atoms, (C₁-C₄)alkoxy optionally substituted with from one to three fluorine atoms, cyano, nitro, amino, (C₁-C₄)alkylamino, di-[(C₁-C₄)alkyl]amino, NR⁵(C=O)(C₁-C₄)alkyl wherein R⁵ is hydrogen or (C₁-C₆)alkyl, and SOₚ(C₁-C₆)alkyl wherein p is zero, one or two; and
with the proviso that: (a) no more than one of NR¹R², CR³R⁴ and R²NCR³ can form a ring; and (b) at least one X must be other than hydrogen when (i) R³ and R⁴ are both hydrogen, (ii) R¹ and R² are selected, independently, from hydrogen and (C₁-C₄)alkyl, and (iii) ring B is mono- or disubstituted with, respectively, one or two halo groups;
or a pharmaceutically acceptable salt thereof.

4. A combination according to any one of claims 1-2, wherein the (SRI) anxiolytic agent or antidepressant is selected from compounds of the formula II: 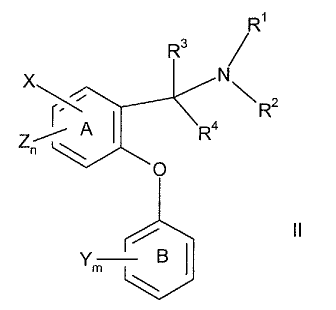 wherein
phenyl ring A and phenyl ring B can each, independently, be replaced by a naphthyl group, and wherein when phenyl ring A is replaced by a naphthyl group, the ethereal oxygen of formula II and the carbon to which R³, R⁴ and NR¹R² are attached, are attached to adjacent ring carbon atoms of the naphthyl group and neither of said adjacent ring carbon atoms is also adjacent to a fused ring carbon atom of said naphthyl group;
n and m are, selected, independently, from one, two and three;
R¹ and R² are selected, independently, from hydrogen, (C₁-C₄)alkyl, (C₂-C₄)alkenyl, and (C₂-C₄)alkynyl, or R¹ and R², together with the nitrogen to which they are attached, form a four to eight membered saturated ring containing one or two heteroatoms, including the nitrogen to which R¹ and R² are attached, wherein the second heteroatom, when present, is selected from oxygen, nitrogen and sulfur, and wherein said ring may optionally be substituted at available binding sites with from one to three substituents selected, independently, from hydroxy and (C₁-C₆)alkyl;
R³ and R⁴ are selected, independently, from hydrogen and (C₁-C₄) alkyl optionally substituted with from one to three fluorine atoms, or R³ and R⁴, together with the carbon to which they are attached, form a four to eight membered saturated carbocyclic ring, and wherein said ring may optionally be substituted at available binding sites with from one to three substituents selected, independently, from hydroxy and (C₁-C₆)alkyl;
or R² and R³, together with the nitrogen to which R² is attached and the carbon to which R³ is attached, form a four to eight membered saturated ring containing one or two heteroatoms, including the nitrogen to which R² is attached, wherein the second heteroatom, when present, is selected from oxygen, nitrogen and sulfur, and wherein said ring may optionally be substituted at available binding sites with from one to three substituents selected, independently, from hydroxy and (C₁-C₆)alkyl;
each X is selected, independently, from phenyl, heteroaryl and heterocycle, and wherein each X may be further substituted by hydrogen, halo, (C₁-C₄)alkyl optionally substituted with from one to three fluorine atoms, (C₁-C₄)alkoxy optionally substituted with from one to three fluorine atoms, cyano, nitro, amino, hydroxy, carbonyl, (C₁-C₄)alkylamino, di-[(C₁-C₄)alkyl]amino, NR⁵(C=O)(C₁-C₄)alkyl, SO₂NR⁵R⁶ and SOₚ(C₁-C₆)alkyl, wherein R⁵ and R⁶ are selected, independently, from hydrogen and (C₁-C₆)alkyl, and p is zero, one or two;
each Y is selected, independently, from hydrogen, halo, (C₁-C₄)alkyl optionally substituted with from one to three fluorine atoms, (C₁-C₄)alkoxy optionally substituted with from one to three fluorine atoms, cyano, nitro, amino, (C₁-C₄)alkylamino, di-[(C₁-C₄)alkyl]amino, NR⁵(C=O)(C₁-C₄)alkyl, SO₂NR⁵R⁶ and SOₚ(C₁-C₆)alkyl, wherein R⁵ and R⁶ are selected, independently, from hydrogen and (C₁-C₆)alkyl, and p is zero, one or two; and
each Z is selected independently from hydrogen, halo, (C₁-C₄)alkyl optionally substituted with from one to three fluorine atoms, (C₁-C₄)alkoxy;
or a pharmaceutically acceptable salt thereof.

5. A combination according to any one of claims 1-4 wherein the neurotransmitter-inducing or precursor agent or a pharmaceutically acceptable salt thereof is selected from:
L-phenylalanine;
L-tyrosine;
L-tryptophan;
L-DOPA; and tyramine.

6. A pharmaceutical composition comprising: (a) a compound that exhibits activity, respectively, as an (SRI) anxiolytic agent or an antidepressant, or a pharmaceutically acceptable salt thereof; (b) a neurotransmitter-inducing or precursor agent or pharmaceutically acceptable salt thereof; and (c) a pharmaceutically acceptable carrier.

7. A pharmaceutical composition according to claim 6 suitable for the treatment of Multiple Sclerosis and other demyelinating conditions in a mammal and wherein the active agents "a" and "b" are present in amounts that render the composition effective in treating, respectively, Multiple Sclerosis and other demyelinating conditions.

8. A pharmaceutical composition according to claim 7 where the demyelinating conditions are encephalomyelitis and peripheral neuropathy, especially painful neuropathy and diabetic neuropathy.

9. A pharmaceutical composition according to any one of claims 6-8, wherein the (SRI) anxiolytic agent or antidepressant is selected from compounds of the formula I, or a pharmaceutically acceptable salt thereof, as defined in claim 3.

10. A pharmaceutical composition according to any one of claims 6-8, wherein the (SRI) anxiolytic agent or antidepressant is selected from compounds of the formula II or a pharmaceutically acceptable salt thereof, as defined in claim 4.

11. A pharmaceutical composition according to any one of claims 6-10 wherein the neuro-transmitter-inducing or precursor agent or a pharmaceutically acceptable salt thereof is selected from:
L-phenylalanine;
L-tyrosine;
L-tryptophan;
L-DOPA; and
Tyramine.

12. A pharmaceutical composition according to any one of claims 6-11 wherein the amount of the (SRI) anxiolytic agent or antidepressant, or pharmaceutically acceptable salt thereof, in said composition is from about 0.05 mg to about 500 mg and the amount of the neurotransmitter-inducing or precursor agent or pharmaceutically acceptable salt thereof is from about 0.05 mg to about 500 mg.

13. Use of (a) a compound that exhibits activity, respectively, as an (SRI) anxiolytic agent or an antidepressant, or a pharmaceutically acceptable salt thereof; in the manufacture of a medicament combined with (b) a neurotransmitter-inducing or precursor agent or pharmaceutically acceptable salt thereof for the treatment of Multiple Sclerosis and other demyelinating disorders.

14. Use according to claim 13 wherein the active agents "a" and "b" above are present in amounts that render the combination of the two agents effective in treating Multiple Sclerosis and other demyelinating diseases.

15. Use according to claim 13 or 14 demyelinating diseases. are encephalomyelitis and peripheral neuropathy, especially diabetic neuropathy and painful neuropathy.

16. Use according to any one of claims 13- 15, wherein the (SRI) anxiolytic agent or antidepressant is selected from compounds of the formula I, or a pharmaceutically acceptable salt thereof, as defined in claim 3.

17. The use according to any one of claims 13- 15, wherein the (SRI) anxiolytic agent or antidepressant is selected from compounds of the formula II, or a pharmaceutically acceptable salt thereof, as defined in 4.

18. The use according to any one of claims 13-17, wherein the (SRI) antianxiety agent or the (SRI) antidepressant, or pharmaceutically acceptable salt thereof, and the neurotransmitter-inducing or precursor agent or pharmaceutically acceptable salt thereof, are administered as part of the same dosage form.

19. The use according to any one of claims 13-18, wherein the neurotransmitter-inducing or precursor agent, or pharmaceutically acceptable salt thereof, is administered in an amount from about 100 mg per day to about 5 grams per day, and the (SRI) anxiolytic agent or antidepressant, or pharmaceutically acceptable salt thereof, is administered in an amount from about 0.05 mg day to about 1500 mg per day.

20. The use according to any one of claims 13-19, wherein the neurotransmitter-inducing or precursor agent or pharmaceutically acceptable salt thereof is selected from:
L-phenylalanine;
L-tyrosine;
L-tryptophan;
tyramine; and
L-DOPA.
